# FASCICULE DE BREVET EUROPEEN

(11) **EP 3 307 194 B1**
(45) Date de publication et mention de la délivrance du brevet: **14.08.2024**
(21) Numéro de dépôt: 16739208.3
(22) Date de dépôt: 10.06.2016
(51) Int. Cl.: A61B 34/30, A61M 25/01

(54) **MODULE ROBOTISE D'ENTRAINEMENT DE CATHETER ET DE GUIDE DE CATHETER**
ROBOTERMODUL ZUR ANSTEUERUNG EINES KATHETERS UND EINER KATHETERFÜHRUNG
ROBOTIC MODULE FOR DRIVING A CATHETER AND A CATHETER GUIDE

(30) Priorité: 12.06.2015 FR 1555365
(43) Date de publication de la demande: 18.04.2018
(73) Titulaire: Robocath, 76000 Rouen (FR)
(72) Inventeur: DEBOEUF, Sébastien, 76240 Bonsecours (FR); DESTREBECQ, Fabien, 27520 Bourgtheroulde (FR); FOURNIER, Bruno, 41100 Saint Ouen (FR); BENCTEUX, Philippe, 76160 St Martin Du Vivier (FR)
(74) Mandataire: Plasseraud IP
(86) Numéro de dépôt international: PCT/FR2016/051399
(87) Numéro de publication internationale: WO 2016/198799

(56) Documents cités:
- EP-A1- 2 875 792
- WO-A1-2014/062890
- US-A1- 2013 035 537
- US-A1- 2014 276 389
- US-A1- 2014 277 334

## Description

La présente invention est relative aux Modules robotisés d'entraînement de cathéter et/ou de guide de cathéter.

L'insertion manuelle d'un cathéter et/ou d'un guide dans un patient est un acte chirurgical relativement classique. Toutefois, cet acte étant monitoré sous rayons X, le chirurgien en charge de cet acte est soumis à une irradiation conséquente s'il réalise une telle opération sur de nombreux patients.

Afin de réduire les risques pour le chirurgien, on tente de robotiser une telle insertion. Cette robotisation est complexe, car la préhension du cathéter est difficile. Celui-ci baigne en effet dans du liquide de conservation et doit rester stérile. La fiabilité de ces systèmes robotisés est un critère déterminant.

Récemment, il a été proposé dans US 7,927,310 un système d'entraînement gérant à la fois la translation et la rotation du cathéter. Le cathéter est maintenu sur une plaquette rotative par rapport à une base pour l'entraînement en rotation. La plaquette elle-même comporte un mécanisme d'entraînement en translation. De plus, on fait appel à des moteurs déportés, à demeure sur le bâti, et à des systèmes de transfert du mouvement jusqu'au cathéter. En effet, on préfère ne pas avoir les moteurs embarqués, pour des raisons d'alimentation en puissance, d'encombrement et de stérilité.

Cette configuration propose donc un premier mode de fonctionnement dans lequel le cathéter et le guide de cathéter vont pouvoir avancer en translation.

Cette configuration propose donc également un deuxième mode de fonctionnement dans lequel le cathéter et le guide de cathéter vont pouvoir tourner sur eux-mêmes dans un même sens, le sens de rotation choisi pouvant être soit le sens horaire soit le sens antihoraire.

Cependant, dans certains passages du système de circulation sanguine du corps humain, comme par exemple un embranchement de veines ou d'artères ou bien comme par exemple une lésion, le passage du cathéter et a fortiori le passage du guide du cathéter qui le précède vont être difficiles et risquent de buter contre une paroi de vaisseau sanguin voire de s'accrocher et d'endommager une paroi de vaisseau sanguin ou encore de prendre un mauvais vaisseau sanguin au niveau d'un embranchement.

Pour pallier cette difficulté, l'invention propose l'ajout d'un troisième mode de fonctionnement dans lequel une translation lente est associée à une rotation alternée rapide du guide de cathéter pour lui permettre de passer la zone sensible sans encombre. Cette translation lente associée à une rotation alternée rapide peut être effectuée pour le guide seul, pour le cathéter seul, pour le guide et le cathéter. Même si le guide et le cathéter sont entraînés, il est possible de n'appliquer ce troisième mode de fonctionnement qu'au guide ou qu'au cathéter.

A cet effet, selon un exemple non revendiqué on prévoit un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter,
- un deuxième mode de fonctionnement dans lequel l'ensemble d'organes d'entraînement fait tourner le guide et/ou le cathéter autour de lui-même,
caractérisé en ce que le procédé comprend également :
- un troisième mode de fonctionnement dans lequel l'ensemble d'organes d'entraînement fait, simultanément, avancer en translation le guide et/ou le cathéter et tourner le guide et/ou le cathéter autour de lui-même alternativement dans un sens puis dans l'autre sens.

A cet effet, selon l'invention on prévoit aussi un module robotisé d'entraînement de cathéter ou de guide, ou bien de cathéter et de guide, comprenant un ensemble d'organes d'entraînement structuré et disposé de façon à pouvoir être piloté de manière à réaliser le procédé selon l'une quelconque des revendications précédentes.

A cet effet, selon l'invention on prévoit encore un module robotisé d'entraînement de cathéter ou de guide, ou bien de cathéter et de guide, comprenant un ensemble d'organes d'entraînement structuré et disposé de façon à pouvoir être piloté :
- dans un premier mode de fonctionnement, de manière à faire avancer en translation le guide et/ou le cathéter,
- dans un deuxième mode de fonctionnement, de manière à faire tourner le guide et/ou le cathéter autour de lui-même,
caractérisé en ce que l'ensemble d'organes d'entraînement est également structuré et disposé de façon à pouvoir être piloté :
- dans un troisième mode de fonctionnement, de manière à simultanément faire avancer en translation le guide et/ou le cathéter tout en le faisant tourner autour de lui-même alternativement dans un sens puis dans l'autre sens.

Dans le troisième mode de fonctionnement, l'ensemble d'organes d'entraînement fait, simultanément, avancer en translation le guide et/ou le cathéter en fonction des variations de la commande d'une interface homme machine et automatiquement tourner le guide et/ou le cathéter autour de lui-même alternativement dans un sens puis dans l'autre sens. Ainsi, l'ergonomie pour le praticien est améliorée tout en offrant une bonne efficacité de progression du guide de cathéter, et ceci sans danger d'accrochage de paroi de vaisseau sanguin pour le patient.

Dans le troisième mode de fonctionnement, l'ensemble d'organes d'entraînement fait, simultanément, avancer en translation le guide et/ou le cathéter en fonction des variations de la commande d'une interface homme machine et automatiquement tourner le guide et/ou le cathéter autour de lui-même alternativement dans un sens puis dans l'autre sens, la fréquence de rotation alternée étant proportionnelle à la vitesse de translation.

Dans des modes de réalisation préférés de invention, on peut éventuellement avoir recours en outre L'une et/ou à l'autre des dispositions suivantes.

Dans un premier mode de réalisation préférentiel, pour lequel le praticien conserve une liberté maximale, il est prévu, un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter en fonction des variations de la commande d'une interface homme machine,
- un deuxième mode de fonctionnement dans lequel l'ensemble d'organes d'entraînement fait tourner le guide et/ou le cathéter autour de lui-même en fonction des variations de la commande d'une interface homme machine,
caractérisé en ce que le procédé comprend également :
- un troisième mode de fonctionnement dans lequel l'ensemble d'organes d'entraînement fait, simultanément, avancer en translation le guide et/ou le cathéter et tourner le guide et/ou le cathéter autour de lui-même alternativement dans un sens puis dans l'autre sens, en fonction des variations de la commande d'une interface homme machine.

Dans un deuxième mode de réalisation préférentiel, pour lequel le praticien dispose d'une facilité d'utilisation optimisée, il est prévu, un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait automatiquement avancer en translation le guide et/ou le cathéter,
- un deuxième mode de fonctionnement dans lequel l'ensemble d'organes d'entraînement fait automatiquement tourner le guide et/ou le cathéter autour de lui-même,
caractérisé en ce que le procédé comprend également :
- un troisième mode de fonctionnement dans lequel l'ensemble d'organes d'entraînement fait, automatiquement et simultanément, avancer en translation le guide et/ou le cathéter et tourner le guide et/ou le cathéter autour de lui-même alternativement dans un sens puis dans l'autre sens.

De préférence, dans le troisième mode de fonctionnement, l'ensemble d'organes d'entraînement fait, simultanément, avancer en translation le guide et/ou le cathéter et tourner le guide et/ou le cathéter autour de lui-même alternativement dans un sens puis dans l'autre sens, le rapport entre la fréquence de rotation alternée et la vitesse de translation étant réglable par l'utilisateur du procédé. Cela permet d'adapter, au souhait et à l'habileté de l'utilisateur, le rapport entre d'une part la fréquence de rotation alternée et d'autre part la vitesse de translation, tout en laissant éventuellement l'utilisateur avancer à son rythme selon les difficultés rencontrées avec la vitesse de translation que cet utilisateur va juger utile.

De préférence, dans le troisième mode de fonctionnement, l'avancée en translation du guide et/ou du cathéter est plus lente que dans le premier mode de fonctionnement, tandis que la rotation alternative du guide et/ou du cathéter autour de lui-même est plus rapide que la rotation du guide et/ou du cathéter autour de lui-même dans le deuxième mode de fonctionnement. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, le guide est un fil présentant un bout recourbé, le bout recourbé avançant le long d'une direction parallèle au fil tout en tournant autour de l'axe du fil dans le troisième mode de fonctionnement. Ainsi, le bout recourbé du fil aide à diriger le guide du cathéter dans la bonne direction grâce à une orientation adéquate de ce bout recourbé du fil.

De préférence, le bout recourbé du guide subit au moins deux changements de sens de rotation le temps qu'il avance d'une distance correspondant à la longueur du bout recourbé, de préférence au moins quatre changements de sens de rotation, encore plus de préférence au moins dix changements de sens de rotation. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, le guide et/ou le cathéter subit au moins deux changements de sens de rotation le temps qu'il avance d'une distance correspondant à une longueur de 5mm, de préférence au moins quatre changements de sens de rotation, encore plus de préférence au moins dix changements de sens de rotation. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, dans le troisième mode de fonctionnement, la fréquence de changement de sens de rotation du guide et/ou du cathéter est d'au moins 1 Hz, de préférence d'au moins 3 Hz, encore plus de préférence d'au moins 10 Hz.

De préférence, dans le troisième mode de fonctionnement, la vitesse de translation du guide et/ou du cathéter est d'au plus 10 mm/s, de préférence d'au plus 3 mm/s, encore plus de préférence d'au plus 1 mm/s.

De préférence, le troisième mode est utilisé pour traverser certaines zones d'embranchement dans le système de circulation sanguine du corps humain. Ce troisième mode de fonctionnement est en effet particulièrement efficace pour traverser les zones sensibles ou difficiles du système de circulation sanguine du corps humain.

De préférence, le troisième mode est utilisé pour traverser certaines zones de lésion dans le système de circulation sanguine du corps humain. Ce troisième mode de fonctionnement est en effet particulièrement efficace pour traverser les zones sensibles ou difficiles du système de circulation sanguine du corps humain.

Selon un autre aspect de l'invention, toujours pour aider le guide et/ou le cathéter à passer les zones sensibles, il est prévu un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter,
- un deuxième mode de fonctionnement dans lequel l'ensemble d'organes d'entraînement fait tourner le guide et/ou le cathéter autour de lui-même,
caractérisé en ce que le procédé comprend également :
- un troisième mode de fonctionnement dans lequel l'ensemble d'organes d'entraînement fait, simultanément, d'une part avancer en translation le guide et/ou le cathéter et d'autre part faire successivement alterner une rotation du guide et/ou du cathéter autour de lui-même toujours dans un même sens suivie d'un arrêt de cette rotation.

De préférence, dans le troisième mode de fonctionnement, ladite rotation dure moins longtemps que ledit arrêt.

De préférence, dans le troisième mode de fonctionnement, ladite rotation dure entre 0.05s et 0.2s, de préférence environ 0.1s, ledit arrêt dure entre 0.3s et 1s, de préférence environ 0.5s, ladite vitesse de translation est comprise entre 1mm/s et 5mm/s, valant de préférence environ 3mm/s.

Dans un exemple de réalisation structurelle, il est prévu que l'ensemble d'organes d'entraînement comprend :
- une base,
- une paire d'organes d'entraînement présentant chacun une surface d'entraînement, la paire d'organes d'entraînement étant plaçable alternativement dans une configuration d'entraînement dans laquelle les surfaces d'entraînement des organes d'entraînement de la paire d'organes d'entraînement sont en prise avec le guide et/ou le cathéter à entraîner et disposés de part et d'autre de celui-ci, et dans une configuration libre dans laquelle la surface d'entraînement des organes d'entraînement de la paire d'organes d'entraînement n'est pas en prise avec le guide et/ou le cathéter,
la paire d'organes d'entraînement étant montée mobile par rapport à la base selon un degré de liberté entre une première et une deuxième positions,
- un organe de commande adapté pour commander de manière répétée cyclique un déplacement par rapport à la base des organes d'entraînement de la paire d'organes d'entraînement en configuration d'entraînement de la première à la deuxième position, entraînant ainsi le guide et/ou le cathéter par rapport à la base, et un déplacement par rapport à la base (132) des organes d'entraînement de la paire d'organes d'entraînement en configuration libre de la deuxième à la première position sans entraîner le guide et/ou le cathéter par rapport à la base.

Cette réalisation permet un déroulement simple et efficace du mouvement de translation.

Dans un exemple de réalisation structurelle, il est aussi prévu que la translation des organes d'entraînement par rapport à la base selon une direction transverse à la direction longitudinale locale du guide et/ou du cathéter et en des sens opposés est adaptée pour permettre un roulement du guide et/ou du cathéter sur les surfaces d'entraînement autour de la direction longitudinale locale du guide et/ou du cathéter.

Cette réalisation permet un déroulement simple et efficace du mouvement de rotation.

Dans un exemple de réalisation structurelle, il est encore prévu que la base est une première base, la paire d'organes d'entraînement est une première paire d'organes d'entraînement, le module robotisé comprenant en outre :
- une deuxième base,
- une deuxième paire d'organes d'entraînement présentant chacun une surface d'entraînement, la deuxième paire d'organes d'entraînement étant plaçable alternativement dans une configuration d'entraînement dans laquelle les surfaces d'entraînement des organes d'entraînement de la deuxième paire d'organes d'entraînement sont en prise avec le guide et/ou le cathéter à entraîner et disposés de part et d'autre de celui-ci, et dans une configuration libre dans laquelle la surface d'entraînement des organes d'entraînement de la deuxième paire d'organes d'entraînement n'est pas en prise avec le guide et/ou le cathéter,
la deuxième paire d'organes d'entraînement étant montée mobile par rapport à la deuxième base selon un degré de liberté entre une première et une deuxième positions,
- l'organe de commande étant adapté en outre pour commander de manière répétée cyclique un déplacement par rapport à la base des organes d'entraînement de la deuxième paire d'organes d'entraînement en configuration d'entraînement de la première à la deuxième position, entraînant ainsi le guide et/ou le cathéter par rapport à la deuxième base, et un déplacement par rapport à la deuxième base des organes d'entraînement de la deuxième paire d'organes d'entraînement en configuration libre de la deuxième à la première position sans entraîner le guide et/ou le cathéter par rapport à la deuxième base.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue schématique de côté d'une installation d'artériographie robotisée.
- les figures 2a-2f sont des schémas illustratifs des modes de déplacement des organes à entraîner,
- la figure 3 est une vue schématique en perspective d'une portion d'un module d'entraînement en configuration libre,
- les figures 4a à 4e sont des schémas simplifiés illustrant un cycle d'entraînement en translation du cathéter selon un mode de réalisation,
- les figures 5a à 5e sont des schémas simplifiés illustrant un cycle d'entraînement en rotation du cathéter selon un mode de réalisation,
- les figures 6a à 6f sont des schémas simplifiés illustrant un cycle d'entraînement en translation du cathéter selon un mode de réalisation,

Sur les différentes figures, les mêmes références désignent des éléments identiques ou similaires. On emploiera indifféremment les expressions extrémité recourbée et bout recourbé pour le guide de cathéter, sauf mention contraire.

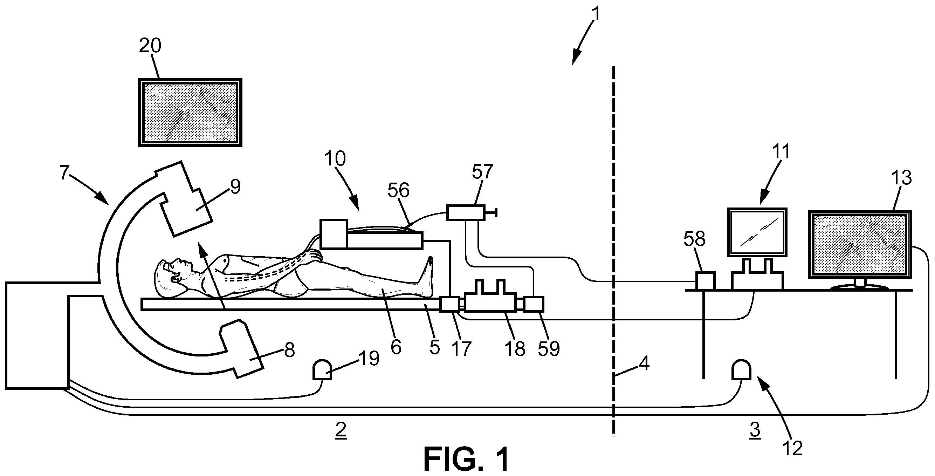

La figure 1 représente schématiquement une installation d'artériographie 1. L'installation d'artériographie 1 est divisée en deux endroits distincts, une salle d'opérations 2 et une salle de commande 3. La salle de commande 3 peut être proche de la salle d'opération 2, séparée de celle-ci par une simple paroi 4, opaque aux rayons X, ou distante. Les matériels de la salle d'opération 2 et de la salle de commande 3 sont reliés entre eux de manière fonctionnelle, par voie filaire, sans fil ou réseau, ....

La salle d'opérations 2 comprend une table d'opérations 5 recevant un patient 6. La salle d'opérations 2 peut également comprendre un imageur médical 7, notamment un imageur par rayons X, comprenant une source 8 et un détecteur 9 disposés de part et d'autre du patient, éventuellement mobiles par rapport au patient.

L'installation d'artériographie 1 comprend un robot 10 disposé dans la salle d'opérations 2.

L'installation d'artériographie 1 comprend un poste de commande 11 disposé dans la salle de commande 3. Le poste de commande 11 est adapté pour commander à distance le robot 10. L'installation d'artériographie 1 peut également comprendre, disposée dans la salle de commande 3, une ou plusieurs commandes distantes 12 de l'imageur 7, communiquant avec l'imageur 7 pour commander celui-ci à distance. L'installation d'artériographie 1 peut également comprendre, disposé dans la salle de commande 3, un écran 13, communiquant avec l'imageur 7, pour visualiser en temps réel dans la salle de commande 3 les images acquises par l'imageur 7.

Le robot 10 peut comprendre un récipient adapté pour contenir un organe médical souple allongé 15 à introduire dans le corps d'un patient. A titre d'organe médical souple allongé 15, il peut par exemple s'agir d'un organe à introduire dans un canal d'un patient, et à déplacer dans ce canal, notamment une artère ou une veine d'un patient, à travers un désilet ménageant une ouverture d'accès dans le patient. L'organe médical souple allongé peut être notamment un cathéter. En variante, l'organe médical souple allongé peut être un guide pour cathéter. Un guide est généralement de diamètre transversal inférieur à celui du cathéter, qui est généralement creux sur une portion proche du patient, voire sur la totalité de sa longueur, de sorte que le guide puisse se déplacer à l'intérieur de celui-ci, notamment à l'intérieur du corps du patient. Le guide peut également comporter une extrémité recourbée, comme il sera décrit plus en détail ci-après.

Le robot 10 peut comprendre un module d'entraînement de l'organe médical souple allongé 15. Le module d'entraînement est commandable à partir du poste de commande 11 pour entraîner l'organe médical souple allongé par rapport au patient selon au moins un degré de liberté, comme ce sera décrit en détails par la suite. Le module d'entraînement peut comprendre un boîtier de communication 17 servant à l'interfaçage avec le poste de commande 11. Au besoin, le robot 10 peut comprendre un boîtier de commande 18 en local, destiné à commander le robot depuis la salle d'opérations 2 si nécessaire.

On notera d'ailleurs que toutes les commandes et les retours disponibles dans la salle de commande 3 peuvent être également disponibles dans la salle d'opérations 2 en vue d'une opération en local, comme par exemple une commande 19 de l'imageur et un écran 20 permettant de visualiser les images acquises par l'imageur 7.

L'organe médical souple allongé 15 creux peut être raccordé à un raccord 56 permettant l'injection d'un produit de contraste facilitant l'imagerie à l'intérieur de l'organe médical souple allongé. L'installation d'artériographie peut comprendre un injecteur 57 de produit de contraste raccordé au raccord 56, commandable par une commande 58 disposée dans la salle de commande 3. Une commande 59 de l'injecteur de produit de contraste peut également être présente en local dans la salle d'opérations 2.

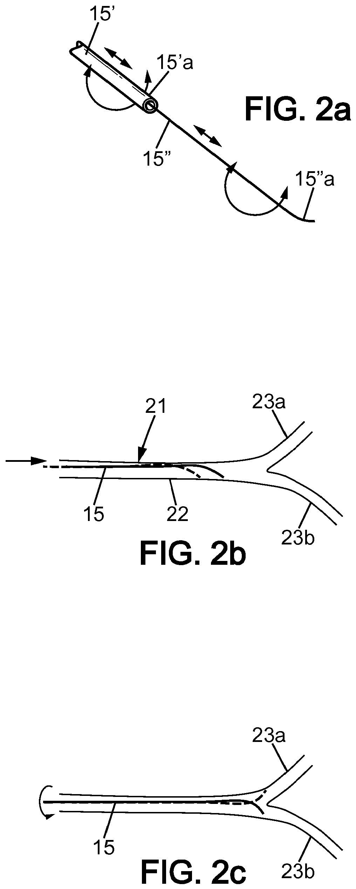

La figure 2a représente les divers degrés de liberté envisageables avec le présent système. On visualise le guide 15'' avec son extrémité avant 15''a légèrement courbée par rapport à l'axe longitudinal principal du guide, et débouchant par l'extrémité avant 15'a du cathéter 15'. Le cathéter 15' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le cathéter 15' peut être soumis à un mouvement combiné des deux mouvements simples décrits ci-dessus.

Le cas échéant, le cathéter 15' peut être soumis à deux mouvements combinés des deux mouvements simples décrits ci-dessus, selon des combinaisons différentes.

Ce qui a été décrit ci-dessus concernant le cathéter s'applique également au guide.

Dans certains cas, le cathéter est lui-même pourvu d'une extrémité courbe, soit pour permettre la navigation sur le même principe qu'un guide, soit pour faciliter le positionnement dans une zone anatomique présentant une courbure particulière.

Sur la figure 2b, on a représenté une artère 21 d'un patient comprenant un tronc principal 22 et deux branches 23a, 23b débouchant sur le tronc principal. La figure 2b illustre le déplacement d'un organe médical souple allongé 15 (ici un guide 15'') selon une translation entre une position reculée représentée en pointillés et une position avancée représentée en traits pleins. Sur la figure 2c, dans la même artère, on a représenté une rotation de l'organe médical souple allongé 15 entre une première position, représenté en pointillés, où l'organe médical souple allongé est prêt à être soumis à une translation en direction de la branche 23a, et une deuxième position, représentée en traits pleins, où l'organe médical souple allongé est prêt à être soumis à une translation en direction de la branche 23b.

L'organe médical souple allongé peut être entraîné selon le ou les déplacements décrits ci-dessus par des organes d'entraînement. Les organes d'entraînement peuvent être agencés par paires.

Sur les figures 2a à 2c ont été représentés des mouvements de translation et des mouvements de rotation correspondant aux premier et deuxième modes de fonctionnement.

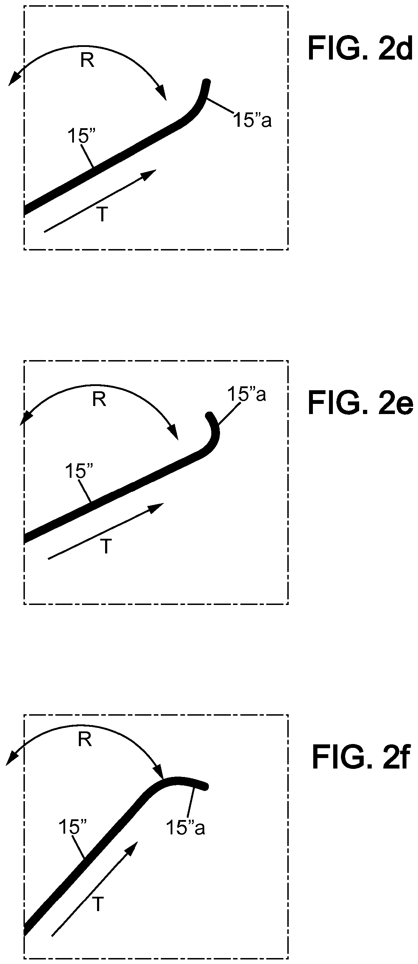

Maintenant en liaison avec les figures 2d à 2f, va être présenté le troisième mode de fonctionnement.

Le guide 15'' de cathéter et son bout recourbé 15''a progressent en translation T le long du guide 15", tandis que, simultanément, le guide 15'' de cathéter et son bout recourbé 15"a subissent une rotation R alternée autour de l'axe du guide 15'' de cathéter.

Les trois figures 2d, 2e et 2f, représentent le bout recourbé 15''a dans différentes positions de d'orientation angulaire lors de la rotation alternée R.

La vitesse de translation T est relativement lente, tandis que la fréquence de rotation alternée R est relativement élevée. Ce troisième mode de fonctionnement, de type translation lente avec rotation alternée rapide simultanée, permet au guide 15''a de cathéter de passer aisément les zones sensibles ou difficiles dans la circulation sanguine de corps humain. C'est le caractère rotation rapide sur une faible course de translation qui permet le passage de la zone délicate sans encombre et sans risque d'accrochage dans la paroi d'un vaisseau sanguin du patient.

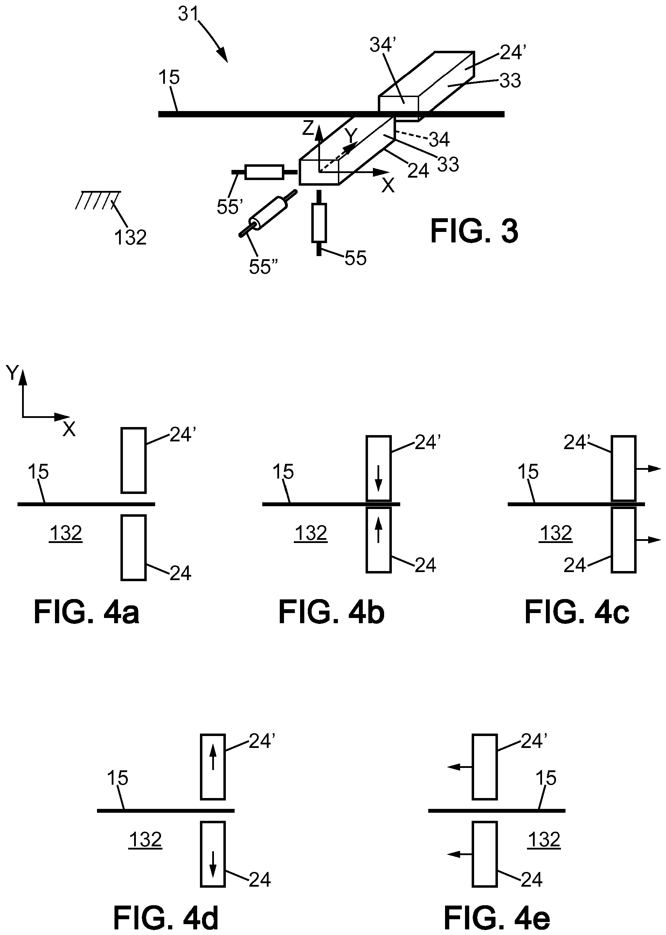

Sur la figure 3, on a représenté un module d'entraînement 131 selon un premier mode de réalisation. Ce module d'entraînement 131 est adapté pour entraîner un organe médical souple allongé 15 s'étendant selon une direction longitudinale X. On notera que la direction longitudinale X au niveau du module d'entraînement 131 n'est pas forcément la même que celle de l'organe médical souple allongé 15 au niveau de son extrémité mais qu'une translation et/ou une rotation de l'organe médical souple allongé 15 selon/autour de la direction longitudinale X au niveau du module d'entraînement 131 entraînera une translation et/ou une rotation de l'organe médical souple allongé 15, respectivement, selon/autour sa direction longitudinale au niveau de son extrémité.

Le module d'entraînement 131 comprend une base 132 et au moins un organe d'entraînement 24 monté mobile par rapport à la base 132. L'organe d'entraînement 24 est par exemple monté mobile par rapport à la base 132.

Dans l'exemple présenté, le module d'entraînement 131 comprend en outre un deuxième organe d'entraînement 24'. L'organe d'entraînement 24, aussi appelé par la suite premier organe d'entraînement, et le deuxième organe d'entraînement 24' forment ensemble une paire d'organes d'entraînement 33. Une paire d'organes d'entraînement 33 comprend deux organes d'entraînement qui coopèrent ensemble pour générer un mouvement de l'organe médical souple allongé 15 par rapport à la base 132. Dans l'exemple présenté, le deuxième organe d'entraînement 24' est monté mobile par rapport à la base 132. Le deuxième organe d'entraînement 24' est par exemple monté mobile par rapport à la base 132.

Le premier organe d'entraînement 24 et le deuxième organe d'entraînement 24' sont appariés pour des mouvements simultanés. Par exemple, les premier et deuxième organes d'entraînement 24, 24' peuvent être commandés individuellement indépendamment l'un de l'autre, mais selon des commandes respectives synchronisées. En variante, on peut prévoir une commande commune qui va être distribuée à l'un et à l'autre des premier et deuxième organes d'entraînement 24, 24' par une liaison mécanique ou électronique entre leurs systèmes de commande.

Chaque organe d'entraînement 24, 24' comporte une surface d'entraînement 34, 34' respectivement. L'organe médical souple allongé 15 est disposé entre les surfaces d'entraînement 34, 34' des organes d'entraînement 24, 24' d'une même paire. Pour fixer les idées, les surfaces d'entraînement 34, 34' sont espacées l'une de l'autre selon la direction Y.

La paire d'organes d'entraînement 24, 24' peut être placée dans une configuration libre, représentée sur la figure 3, dans laquelle la surface d'entraînement 34, 34' des organes d'entraînement 24, 24' de la paire d'organes d'entraînement 33 n'est pas en prise avec l'organe médical souple allongé 15.

La paire d'organes d'entraînement 33 est plaçable dans une configuration d'entraînement dans laquelle les surfaces d'entraînement 34, 34' des organes d'entraînement de la paire d'organes d'entraînement sont en prise avec l'organe médical souple allongé 15 à entraîner. La force appliquée par un organe d'entraînement sur l'organe médical souple allongé dans cette configuration est par exemple de l'ordre de quelques Newtons (5-30 N par exemple). Les moyens de rappel, décrits plus haut, sont par exemple agencés pour ramener la paire d'organes d'entraînement en configuration libre, ce qui permet de fournir une fonction sécuritaire, par exemple en cas de rupture d'alimentation électrique.

Pour placer la paire d'organes d'entraînement 33 alternativement dans les configurations libre et d'entraînement, on peut commander un déplacement relatif l'un vers l'autre des deux organes d'entraînement 24, 24'. Ce déplacement peut par exemple être le déplacement d'un organe d'entraînement 24 par rapport à la base, l'autre restant fixe. En variante, les deux organes d'entraînement 24, 24' peuvent tous deux se déplacer l'un vers l'autre par rapport à la base.

Dans l'exemple, on prévoit un déplacement selon la direction Y.

Dans le mode de réalisation présenté, les deux organes d'entraînement 24, 24' sont mobiles par rapport à la base selon un degré de liberté. Ce degré de liberté diffère de celui permettant le placement alternatif des organes d'entraînement entre les positions libre et d'entraînement. On prévoit notamment que les organes d'entraînement 24, 24' sont mobiles par rapport à la base selon un degré de liberté dans leur configuration d'entraînement. Ainsi, le déplacement des organes d'entraînement selon un degré de liberté dans leur configuration d'entraînement génère un déplacement de l'organe médical souple allongé par rapport à la base 132.

Un exemple sera décrit plus en détail ci-après en relation avec les figures 4a à 4e. Cet exemple décrit la génération d'un mouvement de translation de l'organe médical souple allongé selon sa direction longitudinale X.

La position de départ, représentée à la figure 4a, correspond à celle de la figure 3 décrite ci-dessus. Dans un premier temps, on passe de la configuration libre représentée à la figure 4a à la configuration d'entraînement (figure 4b). Selon l'exemple, ce passage se fait par un mouvement en sens opposé des deux organes d'entraînement selon la direction Y. L'amplitude de ce mouvement peut dépendre de l'organe médical souple allongé 15 à entraîner. Un guide, de diamètre inférieur au cathéter, pouvant nécessiter un mouvement de plus grande amplitude que le cathéter à partir d'une même position de départ.

En configuration d'entraînement, on génère un déplacement simultané dans le même sens des organes d'entraînement selon la direction longitudinale X selon un premier sens, ce qui génère un mouvement identique de l'organe médical souple allongé 15 (figure 4c).

On passe de la configuration d'entraînement représentée à la figure 4c à la configuration libre (figure 4d). Selon l'exemple, ce passage se fait par un mouvement en sens opposé des deux organes d'entraînement selon la direction Y, dans le sens opposé au sens pour faire passer les organes d'entraînement de la configuration d'entraînement à la configuration libre.

En configuration libre, on génère un déplacement simultané (ou non) dans le même sens des organes d'entraînement selon la direction longitudinale X selon un deuxième sens opposé au premier sens, ce qui ne génère pas de mouvement de l'organe médical souple allongé 15 (figure 4e). On est alors revenu à la configuration de départ.

On peut répéter les étapes ci-dessus de manière commandée cyclique pour générer une translation de l'organe médical souple allongé selon une course longue (par exemple de l'ordre de plusieurs mètres) selon la direction longitudinale X dans le premier sens.

Le déplacement de l'organe médical souple allongé selon une course longue selon la direction longitudinale X dans le deuxième sens peut se faire par une suite d'opérations opposées de celle qui vient d'être décrite.

La fréquence du cycle peut être réglable et commandable. En particulier, on peut prévoir une fréquence faible pour l'introduction de l'organe médical souple allongé dans le patient, voire plusieurs niveaux de fréquence faible, pour permettre en particulier une navigation lente dans les environnements difficiles. On peut prévoir une fréquence rapide, par exemple pour un retrait, voire un retrait d'urgence. Les amplitudes de déplacement pour chaque cycle peuvent également être réglables.

Pour la translation, des vitesses comprises entre 0,1 et 200 millimètres par secondes sont envisageables.

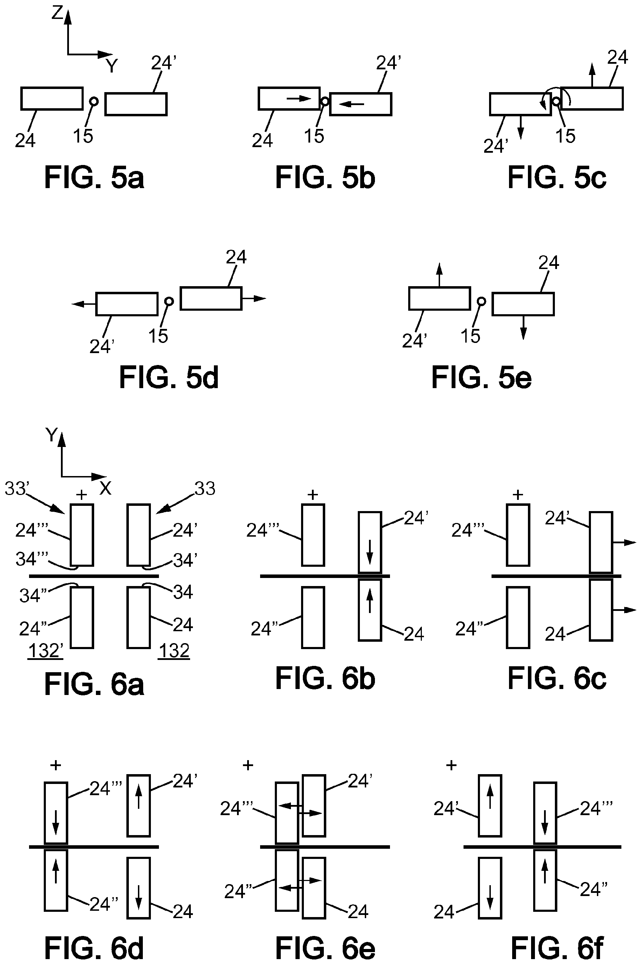

Un exemple sera décrit plus en détail ci-après en relation avec les figures 5a à 5e. Cet exemple décrit la génération d'un mouvement de rotation de l'organe médical souple allongé atour de sa direction longitudinale X.

La position de départ, représentée à la figure 5a, correspond à celle de la figure 3 décrite ci-dessus. Dans un premier temps, on passe de la configuration libre représentée à la figure 5a à la configuration d'entraînement (figure 5b). Selon l'exemple, ce passage se fait par un mouvement en sens opposé des deux organes d'entraînement selon la direction Y. Ce passage est le même que déjà décrit en relation avec les figures 4a, 4b ci-dessus.

En configuration d'entraînement, on génère un déplacement simultané en sens opposé des organes d'entraînement selon une direction Z transversale à la direction longitudinale X, différente de la direction Y, ce qui génère un mouvement de rotation de l'organe médical souple allongé 15 (figure 5c) autour de la direction longitudinale X. En particulier, l'organe médical souple allongé roule, de préférence sans glissement, sur les surfaces d'entraînement 34, 34' des organes d'entraînement 24, 24'. En variante, on pourrait déplacer un seul des deux organes d'entraînement, l'autre restant fixe.

On passe de la configuration d'entraînement représentée à la figure 5c à la configuration libre (figure 5d). Selon l'exemple, ce passage se fait par un mouvement en sens opposé des deux organes d'entraînement selon la direction Y, dans le sens opposé au sens pour faire passer les organes d'entraînement de la configuration d'entraînement à la configuration libre.

En configuration libre, on génère un déplacement simultané (ou non) des organes d'entraînement selon la direction Z, opposé au déplacement décrit ci-dessus en relation avec la figure 5c, ce qui ne génère pas de mouvement de l'organe médical souple allongé 15 (figure 5e). On est alors revenu à la configuration de départ.

On peut répéter les étapes ci-dessus de manière commandée cyclique pour générer une rotation de l'organe médical souple allongé selon une course longue (par exemple de plusieurs fois 360°) autour de la direction longitudinale X dans un premier sens de rotation.

Le déplacement de l'organe médical souple allongé selon une course longue autour de la direction longitudinale X dans le deuxième sens de rotation opposé au premier peut se faire par une suite d'opérations opposées de celle qui vient d'être décrite.

Dans la description ci-dessus, le degré de rotation de l'extrémité libre de l'organe médical souple à l'intérieur du corps du patient peut être surveillé par imagerie. Toutefois, on peut également, en variante ou en complément, chercher à contrôler en amont l'amplitude de la rotation appliquée à l'organe médical souple au niveau du module d'entraînement. Ceci passe par une connaissance du diamètre de l'organe médical souple allongé au niveau des organes d'entraînement 24, 24'. En effet, l'angle de rotation de l'organe médical souple allongé pour un déplacement donné des organes d'actionnement dépend du rapport entre le diamètre de l'organe médical souple allongé et la course des organes d'entraînement. Ce diamètre peut être prédéfini et stocké dans le poste de commande 11. Il suffit d'informer au préalable le poste de commande 11 du type de cathéter utilisé, le type en question comprenant le diamètre. On peut également, en variante, détecter in situ le diamètre de l'organe médical souple allongé. Si la configuration libre de chaque organe d'entraînement constitue une position de référence, on peut connaître la position de l'organe d'entraînement en configuration d'entraînement par exemple en utilisant un système de codage sur l'actionneur associé à chaque organe d'entraînement et permettant de déplacer l'organe d'entraînement de sa configuration libre à sa configuration d'entraînement.

En connaissant la position des deux organes d'entraînement en configuration d'entraînement, et en connaissant l'écart entre les surfaces d'entraînement 34, 34' des deux organes d'entraînement dans leur configuration libre, on peut déterminer l'écart entre les deux surfaces d'entraînement en configuration d'entraînement et, partant, le diamètre de l'organe médical souple allongé.

Cette connaissance peut également être utilisée pour détecter la fin d'un mouvement de retrait de l'organe médical souple allongé. En effet, si le poste de commande 11 détecte une variation soudaine du diamètre détecté au cours du temps lors d'une commande de retrait de l'organe médical souple allongé, cela signifie vraisemblablement que l'organe médical souple allongé a été intégralement retiré du patient, et même du module. Le diamètre détecté alors peut être soit nul, soit par exemple le diamètre du guide si celui-ci s'étend alors entre les deux organes d'entraînement.

On peut également maîtriser le serrage de l'organe médical souple allongé en configuration d'entraînement.

En effet, en configuration d'entraînement, le courant appliqué aux actionneurs est proportionnel à la force de serrage appliquée sur l'organe médical souple allongé. La connaissance de ce courant permet donc de déterminer le serrage appliqué au cathéter. En pratique, on pourra prévoir au niveau du poste de commande 11 différentes consignes en courant pour les actionneurs, disposés dans une plage de serrage acceptable en dehors de laquelle on risquerait soit de voir glisser l'organe médical souple allongé hors de prise, soit de détériorer l'organe médical souple allongé par une trop importante sollicitation mécanique par les organes d'entraînement.

La maîtrise du serrage de l'organe médical souple allongé peut être faite pour n'importe quel mouvement appliqué au cathéter, pas seulement pour le mouvement de rotation décrit ci-dessus.

La détermination du diamètre de l'organe médical souple allongé pourrait être faite pour d'autres mises en oeuvre de déplacement de cathéter que les commandes répétées cycliques décrites ici.

Ainsi, indépendamment des commandes répétées cycliques décrites ici, il semble qu'un mode de réalisation de l'invention se rapporte à un module robotisé d'entraînement d'organe médical souple allongé comprenant :
- une base 132,
- une paire 33 d'organes d'entraînement 24, 24' présentant chacun une surface d'entraînement 34, 34', la paire 33 d'organes d'entraînement 24, 24' étant plaçable par au moins un actionneur 26 dans une configuration d'entraînement dans laquelle les surfaces d'entraînement 34, 34' des organes d'entraînement 24, 24' de la paire 33 d'organes d'entraînement 24, 24' sont en prise avec l'organe médical souple allongé à entraîner et disposés de part et d'autre de celui-ci,
la paire 33 d'organes d'entraînement 24, 24' étant montée mobile par rapport à la base 132 selon un degré de liberté entre une première et une deuxième positions,
- un organe de commande 18, 11 adapté pour commander à partir d'un signal représentatif relatif à l'actionneur 26 (par exemple de manière répétée cyclique) un déplacement par rapport à la base 132 des organes d'entraînement 24, 24' de la paire 33 d'organes d'entraînement 24, 24' en configuration d'entraînement de la première à la deuxième position, entraînant ainsi l'organe médical souple allongé par rapport à la base 132.

En particulier, le signal représentatif relatif à l'actionneur permet de déterminer un écartement entre les surfaces d'entraînement 34, 34', l'organe de commande 18, 11 commandant un déplacement déterminé à partir de l'écartement par rapport à la base 132 des organes d'entraînement 24, 24' de la paire 33 d'organes d'entraînement 24, 24' entraînant ainsi une rotation d'amplitude contrôlée l'organe médical souple allongé par rapport à la base 132.

En particulier, le signal représentatif relatif à l'actionneur permet de maîtriser une force de serrage appliquée à l'organe médical souple allongé dans une gamme admissible de forces de serrage.

Dans les deux exemples de réalisation ci-dessus, on a décrit un déplacement séquencé au cours duquel on attend d'avoir terminé le déplacement d'un organe d'entraînement selon une direction pour entamer un autre déplacement.

Toutefois, étant donné que les actionnements des organes d'entraînement selon divers degrés de liberté peuvent être rendus indépendants en utilisant de manière indépendante les trois systèmes d'actionnement 55, 55', 55'' décrits plus haut, on pourrait mettre en oeuvre de manière simultanée le déplacement d'un organe d'entraînement selon deux degrés de liberté. Par exemple, le déplacement des organes d'entraînement de la position de la figure 5c à celle de la figure 5e pourrait inclure une phase intermédiaire entre une première phase de pur écartement et une deuxième phase de pur retour à la position initiale, où ces deux mouvements sont combinés. Une phase intermédiaire similaire est également envisageable entre la position de la figure 5d et la position de la figure 5b entre la phase de pur retour à la position initiale et une phase de pur rapprochement. En tirant le trait, on pourrait n'avoir plus de phases de pur retour à la position initiale, pur écartement et pur rapprochement, dans la mesure où on ne risque pas de générer de mouvements parasites de l'organe médical souple allongé.

Par ailleurs, alors qu'on a présenté de manière indépendante aux figures 4a-4e un mouvement de translation pure de l'organe médical souple allongé, et aux figures 5a-5e un mouvement de rotation pure, ces deux mouvements pourraient alternativement être combinés. Il suffirait, en configuration en prise, de combiner les mouvements adaptés des organes d'entraînement pour générer simultanément translation et rotation.

L'exemple précédent comprend une unique paire d'organes d'entraînement.

En variante, on pourrait prévoir plusieurs paires d'organes d'entraînement. Par exemple, à titre descriptif, on pourrait prévoir deux paires d'organes d'entraînement. Les organes d'entraînement 24", 24''' de la deuxième paire 33' peuvent être similaires à ceux de la première paire, et en particulier comporter des surfaces d'entraînement 34", 34''', et être actionnés depuis le poste de commande 11 distant, voire le boîtier de commande 18 local selon des mises en oeuvre similaires à celles de la première paire. La première paire 33 et la deuxième paire 33' d'organes d'entraînement peuvent être décalées l'une par rapport à l'autre selon l'axe longitudinal X de l'organe médial souple allongé. Selon un premier exemple, les deux paires 33, 33' peuvent être prévues coplanaires dans leur configuration libre. C'est-à-dire qu'elles peuvent être prévues vis-à-vis d'une base 132 commune aux deux paires. En variante, les bases 132, 132' de chaque paire pourraient être indépendantes, voire non coplanaires.

Les actionnements des deux paires peuvent être synchronisés. Par exemple, les actionnements des deux paires peuvent générer des mouvements identiques simultanés des deux paires.

En variante, les deux paires peuvent être actionnés de manière synchronisées pour générer des mouvements décalés en phase. C'est-à-dire qu'une première paire 33 peut être en configuration d'entraînement pendant qu'une autre paire est en configuration libre, et vice-versa. Par exemple, il y a toujours au moins une paire en configuration d'entraînement. A chaque moment donné, il peut s'agir de la première, de la deuxième, voire des deux en même temps. Une telle configuration permet d'améliorer le maintien de l'organe médical souple allongé. Notamment quand l'organe médical souple allongé est déplacé en frottant contre une zone anatomique du patient, il faut pouvoir assurer un maintien suffisant de celui-ci pour vaincre la résistance locale au déplacement. Ceci est rendu d'autant plus difficile quand l'organe médical souple allongé est glissant, par exemple du fait de son maintien dans une solution.

Un exemple est donné à titre illustratif sur les figures 6a à 6f pour un mode d'entraînement en translation. Sur ces figures, une référence fixe au cours du temps est désignée par le signe « + ». Le mouvement de la première paire, représenté aux figures 6a à 6e a déjà été décrit ci-dessus en relation avec les figures 4a à 4e. La figure 6f représente la même position que la figure 6b, le déplacement étant cyclique.

Les figures 6b à 6f représentent les déplacements de la deuxième paire 33' au cours d'un cycle. Ces déplacements sont déphasés par rapport à ceux de la première paire, la position illustrée sur la figure 6d pour la deuxième paire correspondant à celle de la figure 6b pour la première paire, et ainsi de suite.

Les deux paires sont espacées de manière à éviter toute collision, notamment tel que représenté à la figure 6e, où la deuxième paire est soumise à un déplacement dans le sens de l'avancement de l'organe médical souple allongé et où la première paire est soumise à un déplacement en sens opposé.

A titre illustratif, la figure 6a peut représenter un état initial dans lequel les deux paires se situent à distance de l'organe médical souple allongé. Lors de la mise en route du système, la première paire sera commandée puis, de manière déphasée, la deuxième paire.

Cette mise en oeuvre s'applique pour d'autres mouvements que la translation. Cette mise en oeuvre s'applique pour plus de deux paires. Dans ce cas, les paires sont, le cas échéants, toutes déphasées les unes par rapport aux autres, ou certaines paires peuvent être en phase les unes avec les autres.

## Revendications

1. Module robotisé d'entraînement de cathéter (15') ou de guide (15''), ou bien de cathéter (15') et de guide (15''), comprenant un ensemble d'organes d'entraînement (24, 24', 24", 24‴) structuré et disposé de façon à pouvoir être piloté :
- dans un premier mode de fonctionnement, de manière à faire avancer en translation (T) le guide (15") et/ou le cathéter (15'),
- dans un deuxième mode de fonctionnement, de manière à faire tourner le guide (15'') et/ou le cathéter (15') autour de lui-même,
l'ensemble d'organes d'entraînement (24, 24', 24", 24‴) étant également structuré et disposé de façon à pouvoir être piloté :
- dans un troisième mode de fonctionnement, de manière à simultanément faire avancer en translation (T) le guide (15'') et/ou le cathéter (15') tout en le faisant tourner autour de lui-même,
**caractérisé en ce que** :
- dans le troisième mode de fonctionnement, l'ensemble d'organes d'entraînement (24, 24', 24", 24‴) fait, simultanément, avancer en translation (T) le guide (15'') et/ou le cathéter (15') en fonction des variations de la commande d'une interface homme machine et automatiquement tourner le guide (15") et/ou le cathéter (15') autour de lui-même alternativement dans un sens puis dans l'autre sens, la fréquence de rotation alternée étant proportionnelle à la vitesse de translation.

2. Module robotisé d'entraînement selon la revendication 1, **caractérisé en ce que** :
- dans le premier mode de fonctionnement, l'ensemble d'organes d'entraînement (24, 24', 24", 24''') fait avancer en translation (T) le guide (15") et/ou le cathéter (15') en fonction des variations de la commande d'une interface homme machine,
- dans le deuxième mode de fonctionnement, l'ensemble d'organes d'entraînement (24, 24', 24", 24''') fait tourner le guide (15") et/ou le cathéter (15') autour de lui-même en fonction des variations de la commande d'une interface homme machine.

3. Module robotisé d'entraînement selon l'une quelconque des revendications précédentes, **caractérisé en ce que** :
- dans le premier mode de fonctionnement, l'ensemble d'organes d'entraînement (24, 24', 24", 24‴) fait automatiquement avancer en translation (T) le guide (15") et/ou le cathéter (15'),
- dans le deuxième mode de fonctionnement, l'ensemble d'organes d'entraînement (24, 24', 24", 24‴) fait automatiquement tourner le guide (15") et/ou le cathéter (15') autour de lui-même.

4. Module robotisé d'entraînement selon l'une quelconque des revendications précédentes, **caractérisé en ce que** :
- dans le troisième mode de fonctionnement, l'ensemble d'organes d'entraînement (24, 24', 24", 24‴) fait, simultanément, avancer en translation (T) le guide (15") et/ou le cathéter (15') et tourner le guide (15") et/ou le cathéter (15') autour de lui-même alternativement dans un sens puis dans l'autre sens, le rapport entre la fréquence de rotation alternée et la vitesse de translation étant réglable par l'utilisateur du procédé.

5. Module robotisé d'entraînement selon l'une quelconque des revendications précédentes, **caractérisé en ce que** :
- dans le troisième mode de fonctionnement, l'avancée en translation (T) du guide (15'') et/ou du cathéter (15') est plus lente que dans le premier mode de fonctionnement, tandis que la rotation (R) alternative du guide (15'') et/ou du cathéter (15') autour de lui-même est plus rapide que la rotation (R) du guide (15") et/ou du cathéter (15') autour de lui-même dans le deuxième mode de fonctionnement.

6. Module robotisé d'entraînement selon l'une quelconque des revendications précédentes, **caractérisé en ce que** :
- le guide (15'') est un fil présentant un bout recourbé (15"a), le bout recourbé (15"a) avançant le long d'une direction parallèle au fil tout en tournant autour de l'axe du fil dans le troisième mode de fonctionnement,
- de préférence, le bout recourbé (15''a) du guide (15'') subit au moins deux changements de sens de rotation (R) le temps qu'il avance d'une distance correspondant à la longueur du bout recourbé (15" a), de préférence au moins quatre changements de sens de rotation (R), encore plus de préférence au moins dix changements de sens de rotation (R).

7. Module robotisé d'entraînement selon l'une quelconque des revendications précédentes, **caractérisé en ce que** :
- le guide (15") et/ou le cathéter (15') subit au moins deux changements de sens de rotation (R) le temps qu'il avance d'une distance correspondant à une longueur de 5mm, de préférence au moins quatre changements de sens de rotation (R), encore plus de préférence au moins dix changements de sens de rotation (R),
- et/ou dans le troisième mode de fonctionnement, la fréquence de changement de sens de rotation (R) du guide (15") et/ou du cathéter (15') est d'au moins 1 Hz, de préférence d'au moins 3 Hz, encore plus de préférence d'au moins 10 Hz,
- et/ou dans le troisième mode de fonctionnement, la vitesse de translation (T) du guide (15") et/ou du cathéter (15') est d'au plus 10 mm/s, de préférence d'au plus 3 mm/s, encore plus de préférence d'au plus 1 mm/s.

8. Module robotisé d'entraînement selon l'une quelconque des revendications précédentes, **caractérisé en ce que** l'ensemble d'organes d'entraînement (24, 24', 24", 24''') comprend :
- une base (132),
- une paire (33) d'organes d'entraînement (24, 24') présentant chacun une surface d'entraînement (34, 34'), la paire (33) d'organes d'entraînement (24, 24') étant plaçable alternativement dans une configuration d'entraînement dans laquelle les surfaces d'entraînement (34, 34') des organes d'entraînement (24, 24') de la paire (33) d'organes d'entraînement (24, 24') sont en prise avec le guide (15") et/ou le cathéter (15') à entraîner et disposés de part et d'autre de celui-ci, et dans une configuration libre dans laquelle la surface d'entraînement (34, 34') des organes d'entraînement (24, 24') de la paire (33) d'organes d'entraînement (24, 24') n'est pas en prise avec le guide (15") et/ou le cathéter (15'),
la paire (33) d'organes d'entraînement (24, 24') étant montée mobile par rapport à la base (132) selon un degré de liberté entre une première et une deuxième positions,
- un organe de commande (18, 11) adapté pour commander de manière répétée cyclique un déplacement par rapport à la base (132) des organes d'entraînement (24, 24') de la paire (33) d'organes d'entraînement (24, 24') en configuration d'entraînement de la première à la deuxième position, entraînant ainsi le guide (15'') et/ou le cathéter (15') par rapport à la base (132), et un déplacement par rapport à la base (132) des organes d'entraînement (24, 24') de la paire (33) d'organes d'entraînement (24, 24') en configuration libre de la deuxième à la première position sans entraîner le guide (15") et/ou le cathéter (15') par rapport à la base,
de préférence, dans lequel la translation (T) des organes d'entraînement (24, 24') par rapport à la base (132) selon une direction (Z) transverse à la direction longitudinale (X) locale du guide (15") et/ou du cathéter (15') et en des sens opposés est adaptée pour permettre un roulement du guide (15") et/ou du cathéter (15') sur les surfaces d'entraînement (34, 34') autour de la direction longitudinale (X) locale du guide (15") et/ou du cathéter (15').

9. Module robotisé d'entraînement selon la revendication 8, dans lequel la base (132) est une première base, la paire (33) d'organes d'entraînement (24, 24') est une première paire d'organes d'entraînement, le module robotisé comprenant en outre :
- une deuxième base (132'),
- une deuxième paire (33') d'organes d'entraînement (24", 24‴) présentant chacun une surface d'entraînement (34'', 34‴), la deuxième paire (33') d'organes d'entraînement (24'', 24‴) étant plaçable alternativement dans une configuration d'entraînement dans laquelle les surfaces d'entraînement (34'', 34‴) des organes d'entraînement (24", 24''') de la deuxième paire (33') d'organes d'entraînement (24", 24‴) sont en prise avec le guide (15") et/ou le cathéter (15') à entraîner et disposés de part et d'autre de celui-ci, et dans une configuration libre dans laquelle la surface d'entraînement (34", 34‴) des organes d'entraînement (24", 24‴) de la deuxième paire (33') d'organes d'entraînement (24", 24‴) n'est pas en prise avec le guide (15") et/ou le cathéter (15'),
la deuxième paire (33') d'organes d'entraînement (24", 24''') étant montée mobile par rapport à la deuxième base (132') selon un degré de liberté entre une première et une deuxième positions,
- l'organe de commande (18, 11) étant adapté en outre pour commander de manière répétée cyclique un déplacement par rapport à la base (132') des organes d'entraînement (24", 24‴) de la deuxième paire (33') d'organes d'entraînement (24", 24‴) en configuration d'entraînement de la première à la deuxième position, entraînant ainsi le guide (15") et/ou le cathéter (15') par rapport à la deuxième base (132'), et un déplacement par rapport à la deuxième base (132') des organes d'entraînement (24", 24''') de la deuxième paire (33') d'organes d'entraînement (24", 24‴) en configuration libre de la deuxième à la première position sans entraîner le guide (15") et/ou le cathéter (15') par rapport à la deuxième base (32'),
- les actionnements des deux paires (33,33') étant synchronisés.

## Patentansprüche

1. Robotikmodul zum Antrieb eines Katheters (15') oder einer Führung (15") oder eines Katheters (15') und einer Führung (15"), umfassend eine Anordnung von Antriebselementen (24, 24', 24", 24‴), die derart strukturiert und angeordnet sind, dass sie gesteuert werden können:
- in einer ersten Betriebsart, um die Führung (15") und/oder den Katheter (15') translatorisch vorwärts zu bewegen (T),
- in einer zweiten Betriebsart, um die Führung (15") und/oder den Katheter (15') um sich selbst zu drehen,
wobei die Anordnung von Antriebselementen (24, 24', 24", 24‴) ebenfalls derart strukturiert und angeordnet ist, dass er gesteuert werden kann:
- in einer dritten Betriebsart, um gleichzeitig die Führung (15") und/oder den Katheter (15') translatorisch vorwärts zu bewegen (T) und um sich selbst zu drehen,
**dadurch gekennzeichnet, dass**:
- in der dritten Betriebsart die Anordnung von Antriebselementen (24, 24', 24", 24‴) gleichzeitig die Führung (15") und/oder den Katheter (15') entsprechend den Änderungen des Befehls einer Mensch-Maschine-Schnittstelle translatorisch vorwärts bewegt (T) und automatisch die Führung (15") und/oder den Katheter (15') alternativ in einer Richtung und dann in der anderen Richtung um sich selbst dreht, wobei die Frequenz der alternierenden Drehung proportional zur Translationsgeschwindigkeit ist.

2. Robotik-Antriebsmodul nach Anspruch 1, **dadurch gekennzeichnet, dass**:
- in der ersten Betriebsart die Gesamtheit der Antriebselemente (24, 24', 24", 24‴) die Führung (15") und/oder den Katheter (15') entsprechend den Änderungen der Befehle einer Mensch-Maschine-Schnittstelle translatorisch vorwärts bewegt (T),
- in der zweiten Betriebsart die Anordnung von Antriebselementen (24, 24', 24", 24‴) die Führung (15") und/oder den Katheter (15') entsprechend den Änderungen der Befehle einer Mensch-Maschine-Schnittstelle um sich selbst dreht.

3. Robotik-Antriebsmodul nach einem der vorhergehenden Ansprüche, **dadurch gekennzeichnet, dass**:
- in der ersten Betriebsart die Anordnung von Antriebselementen (24, 24', 24", 24‴) automatisch eine Translationsbewegung (T) der Führung (15") und/oder des Katheters (15') bewirkt,
- in der zweiten Betriebsart die Antriebselemente(24, 24', 24", 24‴) automatisch die Führung (15") und/oder der Katheter (15') um sich selbst dreht.

4. Robotik-Antriebsmodul nach einem der vorhergehenden Ansprüche, **dadurch gekennzeichnet, dass**:
- in der dritten Betriebsart die Anordnung von Antriebselementen (24, 24', 24", 24‴) gleichzeitig die Führung (15") und/oder den Katheter (15') translatorisch vorwärts bewegt (T) und die Führung (15") und/oder den Katheter (15') alternativ in einer Richtung und dann in der anderen Richtung um sich selbst dreht, wobei das Verhältnis zwischen der Frequenz der alternierenden Drehung und der Translationsgeschwindigkeit durch den Benutzer des Verfahrens einstellbar ist.

5. Robotik-Antriebsmodul nach einem der vorhergehenden Ansprüche, **dadurch gekennzeichnet, dass**:
- in der dritten Betriebsart die translatorische Vorwärtsbewegung (T) der Führung (15") und/oder des Katheters (15') langsamer ist als in der ersten Betriebsart, während die alterntative Drehung (R) der Führung (15") und/oder des Katheters (15') um sich selbst schneller ist als die Drehung (R) der Führung (15") und/oder des Katheters (15') um sich selbst in der zweiten Betriebsart.

6. Robotik-Antriebsmodul nach einem der vorhergehenden Ansprüche, **dadurch gekennzeichnet, dass**:
- die Führung (15") ein Draht mit einem gebogenen Ende (15"a) ist, wobei sich das gebogene Ende (15"a) entlang einer Richtung parallel zu dem Draht vorwärts bewegt, während es sich in der dritten Betriebsart um die Achse des Drahtes dreht,
- bevorzugt das gebogene Ende (15"a) der Führung (15") wenigstens zwei Änderungen der Drehrichtung (R) erfährt, während es sich um eine Strecke vorwärts bewegt, die der Länge des gebogenen Endes (15"a) entspricht, bevorzugt wenigstens vier Änderungen der Drehrichtung (R), stärker bevorzugt wenigstens zehn Änderungen der Drehrichtung (R).

7. Robotik-Antriebsmodul nach einem der vorhergehenden Ansprüche, **dadurch gekennzeichnet, dass**:
- die Führung (15") und/oder der Katheter (15') wenigstens zwei Änderungen der Drehrichtung (R) erfährt, während sie sich um eine Strecke bewegt, die einer Länge von 5 mm entspricht, bevorzugt wenigstens vier Änderungen der Drehrichtung (R), stärker bevorzugt wenigstens zehn Änderungen der Drehrichtung (R),
- und/oder in der dritten Betriebsart die Frequenz der Änderung der Drehrichtung (R) der Führung (15") und/oder des Katheters (15') wenigstens 1 Hz, bevorzugt wenigstens 3 Hz, stärker bevorzugt wenigstens 10 Hz beträgt,
- und/oder im dritten Betriebsmodus die Translationsgeschwindigkeit (T) der Führung (15") und/oder des Katheters (15') höchstens 10 mm/sec, bevorzugt höchstens 3 mm/sec, stärker bevorzugt höchstens 1 mm/sec beträgt.

8. Robotik-Antriebsmodul nach einem der vorhergehenden Ansprüche, **dadurch gekennzeichnet, dass** die Anordnung von Antriebselementen (24, 24', 24", 24‴) umfasst:
- eine Basis (132),
- ein Paar (33) von Antriebselementen (24, 24') die jeweils eine Antriebsfläche (34, 34') aufweisen, wobei das Paar (33) von Antriebselementen (24, 24') alternativ in eine Antriebskonfiguration gebracht werden kann, in der die Antriebsflächen (34, 34') der Antriebselemente (24, 24') des Paares (33) von Antriebselementen (24, 24') mit der Führung (15") und/oder dem anzutreibenden Katheter (15') in Eingriff stehen und auf beiden Seiten davon angeordnet sind, und in einer freien Konfiguration, in der die Antriebsfläche (34, 34') der Antriebselemente (24, 24') des Paars (33) von Antriebselementen (24, 24') nicht mit der Führung (15") und/oder dem Katheter (15') in Eingriff steht, wobei das Paar (33) von Antriebselementen (24, 24') in Bezug auf die Basis (132) gemäß einem Freiheitsgrad zwischen einer ersten und einer zweiten Position beweglich montiert ist,
- eine Steuereinheit (18, 11), die derart ausgebildet ist, dass sie zyklisch wiederholt eine Bewegung der Antriebselemente (24, 24') des Paars (33) von Antriebselementen in der Antriebskonfiguration von der ersten in die zweite Position relativ zur Basis (132) steuert, wodurch die Führung (15") und/oder der Katheter (15') relativ zur Basis (132) angetrieben werden, und eine Bewegung der Antriebselemente (24, 24') des Paars (33) von Antriebselementen (24, 24') relativ zur Basis (132) steuert, wodurch die Führung (15") und/oder der Katheter (15') relativ zur Basis (132) angetrieben werden, in freier Konfiguration von der zweiten in die erste Position, ohne die Führung (15") und/oder den Katheter (15') relativ zu der Basis anzutreiben, bevorzugt, wobei die Translation (T) der Antriebselemente (24, 24') in Bezug auf die Basis (132) in einer Richtung (Z) quer zur lokalen Längsrichtung (X) der Führung (15") und/oder des Katheters (15') und in entgegengesetzten Richtungen angepasst ist, um ein Rollen der Führung (15") und/oder des Katheters (15') auf den Antriebsflächen (34, 34') um die lokale Längsrichtung (X) der Führung (15") und/oder des Katheters (15') zu ermöglichen.

9. Robotik-Antriebsmodul nach Anspruch 8, wobei die Basis (132) eine erste Basis ist,
das Paar (33) von Antriebselementen (24, 24') ein erstes Paar von Antriebselementen ist, wobei das Robotermodul ferner umfasst:
- eine zweite Basis (132'),
- ein zweites Paar (33') von Antriebselementen (24", 24‴), die jeweils eine Antriebsfläche (34', 34‴) aufweisen, wobei das zweite Paar (33') von Antriebselementen (24", 24‴) alternativ in eine Antriebskonfiguration gebracht werden kann, in der die Antriebsflächen (34" 34' ‴) der Antriebselemente (24", 24‴) des zweiten Paars (33') von Antriebselementen (24", 24‴) mit der Führung (15") und/oder dem anzutreibenden Katheter (15') in Eingriff stehen und auf beiden Seiten davon angeordnet sind, und in einer freien Konfiguration, in der die Antriebsfläche (34‴) der Antriebselemente (24", 24‴) des zweiten Paars (33') von Antriebselementen (24", 24‴) nicht mit der Führung (15") und/oder dem Katheter (15') in Eingriff steht, wobei das zweite Paar (33') von Antriebselementen (24", 24‴) in Bezug auf die zweite Basis (132') gemäß einem Freiheitsgrad zwischen einer ersten und einer zweiten Position beweglich montiert ist,
- das Steuerelement (18, 11) ferner dazu ausgebildet ist, eine Bewegung relativ zur Basis (132') der Antriebselemente (24", 24‴) des zweiten Paars (33') von Antriebselementen (24", 24‴) in der Antriebskonfiguration von der ersten zur zweiten Position zyklisch wiederholt zu steuern, dadurch die Führung (15") und/oder den Katheter (15') relativ zur zweiten Basis (132') anzutreiben und eine Bewegung relativ zur zweiten Basis (132') der Antriebselemente des zweiten Paars (33') von Antriebselementen (24", 24‴) in freier Konfiguration von der zweiten zur ersten Position anzutreiben, ohne die Führung (15") und/oder den Katheter (15') relativ zur zweiten Basis (32') anzutreiben,
- wobei die Betätigungen der beiden Paare (33, 33') synchronisiert sind.

## Claims

1. A robotic module of driving a catheter (15') or a guide (15"), or a catheter (15') and a guide (15"), comprising an assembly of drive members (24, 24', 24", 24‴) structured and arranged so as to be able to be controlled:
- in a first mode of operation, so as to make the guide (15") and/or the catheter (15') advance in translation (T);
- in a second mode of operation, so as to make the guide (15") and/or the catheter (15') rotate around itself;
the assembly of drive members (24, 24', 24", 24‴) being also structured and arranged so as to be able to be controlled:
- in a third mode of operation, so as to simultaneously make the guide (15") and/or the catheter (15') advance in translation (T) while making it rotate around itself **characterized in that**:
- in the third mode of operation, the assembly of drive members (24, 24', 24", 24"') simultaneously makes the guide (15") and/or the catheter (15') advance in translation (T) according to the variations of the command from a man machine interface and rotates the guide (15") and/or the catheter (15') automatically around itself alternately in one direction and then in the other direction, the frequency of alternating rotation being proportional to the speed of translation.

2. The robotic drive module according to claim 1, **characterized in that**:
- in the first mode of operation, the assembly of drive members (24, 24', 24", 24"') makes the guide (15") and/or the catheter (15') advance in translation (T) according to the variations of the command from a man machine interface;
- in the second mode of operation, the assembly of drive members (24, 24', 24", 24"') makes the guide (15") and/or the catheter (15') rotate around itself according to the variations of the command from a man machine interface.

3. The robotic drive module according to any one of the preceding claims, **characterized in that**:
- in the first mode of operation, the assembly of drive members (24, 24', 24", 24"') automatically makes the guide (15") and/or the catheter (15') advance in translation (T);
- in the second mode of operation, the assembly of drive members (24, 24', 24", 24"') automatically makes the guide (15") and/or the catheter (15') rotate around itself.

4. The robotic drive module according to any one of the preceding claims, **characterized in that**:
- in the third mode of operation, the assembly of drive members (24, 24', 24", 24"') simultaneously makes the guide (15") and/or the catheter (15') advance in translation (T) and rotates the guide (15") and/or the catheter (15') around itself alternately in one direction and then in the other direction, the ratio between the frequency of alternating rotation and the speed of translation is adjustable by the user of the method.

5. The robotic drive module according to any one of the preceding claims, **characterized in that**:
- in the third mode of operation, the advance in translation (T) of the guide (15") and/or the catheter (15') is slower than in the first mode of operation, whereas the alternating rotation (R) of the guide (15") and/or the catheter (15') around itself is faster than the rotation (R) of the guide (15") and/or catheter (15') around itself in the second mode of operation.

6. The robotic drive module according to any one of the preceding claims, **characterized in that**:
- the guide (15") is a wire having a bent-back tip (15"a), with the bent-back tip (15"a) advancing along a direction parallel to the wire while rotating around the axis of the wire in the third mode of operation,
- preferably, the bent-back tip (15"a) of the guide (15") undergoes at least two changes of direction of rotation (R) in the time that it advances a distance corresponding to the length of the bent-back tip (15"a), preferably at least four changes of direction of rotation (R), still more preferably at least ten changes of direction of rotation (R).

7. The robotic drive module according to any one of the preceding claims, **characterized in that**:
- the guide (15") and/or the catheter (15') undergoes at least two changes of direction of rotation (R) in the time that it advances a distance corresponding to a length of 5 mm, preferably at least four changes of direction of rotation (R), still more preferably at least ten changes of direction of rotation (R),
- and/or in the third mode of operation, the frequency of changing direction of rotation (R) of the guide (15") and/or the catheter (15') is at least 1 Hz, preferably at least 3 Hz, still more preferably at least 10 Hz,
- and/or in the third mode of operation, the speed of translation (T) of the guide (15") and/or the catheter (15') is it most 10 mm/s, preferably at most 3 mm/s, still more preferably at most 1 mm/s.

8. The robotic drive module according to any one of the preceding claims, **characterized in that** the assembly of drive members (24, 24', 24", 24‴) comprises:
- a base (132);
- a pair (33) of drive members (24, 24') each having a drive surface (34, 34'), the pair (33) of drive members (24, 24') could be placed alternately in a drive configuration in which the drive surfaces (34, 34') of the drive members (24, 24') of the pair (33) of drive members (24, 24') are engaged with the guide (15") and/or the catheter (15') to be driven and arranged on either side thereof, and in a released configuration in which the drive surface (34, 34') of the drive members (24, 24') of the pair (33) of drive members (24, 24') is not engaged with the guide (15") and/or the catheter (15'),
the pair (33) of drive members (24, 24') being mounted movable relative to the base (132) according to a degree of freedom between first and second positions,
- a command member (18, 11) suited for cyclic repeatedly commanding a displacement relative to the base (132) of the drive members (24, 24') of the pair (33) of drive members (24, 24') in the drive configuration from the first to the second position, thereby driving the guide (15") and/or the catheter (15') relative to the base (132), and a displacement relative to the base (132) of the drive members (24, 24') from the pair (33) of drive members (24, 24') in the released configuration from the second to the first position without driving the guide (15") and/or the catheter (15') relative to the base,
preferably, wherein the translation (T) of the drive members (24, 24') relative to the base (132) along a direction (Z) transverse to the local longitudinal direction (X) of the guide (15") and/or the catheter (15') and in opposite directions is adapted for allowing winding of the guide (15") and/or the catheter (15') on the drive surfaces (34, 34') around the local longitudinal direction (X) of the guide (15") and/or the catheter (15').

9. The robotic drive module according to claim 8, wherein the base (132) is a first base, the pair (33) of drive members (24, 24) is a first pair of drive members, the robotic module further comprising:
- a second base (132'),
- a second pair (33') of drive members (24", 24‴) each having a drive surface (34", 34‴), the second pair (33') of drive members (24", 24‴) could be placed alternately in a drive configuration in which the drive surfaces (34", 34‴) of the drive members (24", 24‴) of the second pair (33') of drive members (24", 24‴) are engaged with the guide (15") and/or the catheter (15') to be driven and arranged on either side thereof, and in a released configuration in which the drive surface (34", 34‴) of the drive members (24", 24‴) of the second pair (33') of drive members (24", 24‴) is not engaged with the guide (15") and/or the catheter (15'),
the second pair (33') of drive members (24", 24‴) being mounted movable relative to the second base (132') according to a degree of freedom between first and second position,
- the command member (18, 11) being further suited for cyclic repeatedly commanding a displacement relative to the base (132') of the drive members (24", 24‴) of the second pair (33') of drive members (24", 24‴) in the drive configuration from the first to the second position, thereby driving the guide (15") and/or the catheter (15') relative to the second base (132'), and a displacement relative to the second base (132') of the drive members (24", 24‴) from the second pair (33) of drive members (24", 24‴) in the released configuration from the second to the first position without driving the guide (15") and/or the catheter (15') relative to the second base (32'),
- the actuations of the two pairs (33, 33') being synchronized.
